# EUROPEAN PATENT SPECIFICATION

(11) **EP 1 285 672 B1**
(45) Date of publication and mention of the grant of the patent: **03.10.2007**
(21) Application number: 02255572.6
(22) Date of filing: 08.08.2002
(51) Int. Cl.: A61M 5/00, A61L 2/20, A61B 19/02

(54) **Prefilled injector package and sterilizing or disinfecting method therefor.**
Vorgefüllte Injektionsverpackung und dafür vorgesehenes Verfahren zum Desinfizieren oder Sterilisieren.
Emballage d'injecteur pré-empli et son procédé de stérilisation ou de désinfection

(30) Priority: 10.08.2001 JP 2001243799
(43) Date of publication of application: 26.02.2003
(73) Proprietor: SEIKAGAKU CORPORATION, Chiyoda-ku, Tokyo 100-0005 (JP)
(72) Inventor: Hamai, Akio, Fussa-shi, Tokyo (JP); Miyamoto, Kenji, Kanagawa-ken (JP); Kano, Hiroki, Tokyo (JP); Shea, John J., NC 27949 (US)
(74) Representative: Benson, John Everett

(56) References cited:
- WO-A-94/13328
- GB-A- 1 534 175
- US-A- 3 473 646
- US-A- 6 073 759
- US-A- 6 080 456
- US-B1- 6 228 324

## Description

### BACKGROUND OF THE INVENTION

The present invention relates to a prefilled injector package and a method for sterilizing or disinfecting the prefilled injector package, and more particularly, to a prefilled injector package, e.g. a package enclosing a injector previously filled with a medicine (drug), which is capable of not only conducting simultaneous sterilization or disinfection of an injector body and a medicine previously filled therein by heat-treating the injector package, but also preventing water from depositing onto an injection nozzle or a cap when subjecting the injector package to sterilization using steam or hot water, and a method for sterilizing or disinfecting the prefilled injector package.

Hitherto, a prefilled injector package, i.e., so-called a package enclosing injector previously filled with a medicine (drug), has been produced by aseptically filling a medicine sterilized by a sterile filtration into an injector body; sterilizing or disinfecting the injector body filled with the sterilized medicine; and then aseptically packaging the sterilized or disinfected injector body previously filled with the sterilized medicine, or by packaging the injector body previously filled with the sterilized medicine as described above; and then subjecting the injector body previously filled with the medicine to gas sterilization. Thus, any of the conventional processes for producing the prefilled injector packages has required two sterilization or disinfection treatments, i.e., sterilization of medicine by sterile filtration, and sterilization or disinfection of injector body previously filled with such a sterilized medicine. However, the two treatments are merely applicable to such cases where the medicine previously filled in the injector body can be sterilized by sterile filtration, and are not applicable to highly-viscoelastic fluids (medicines) incapable of sterile filtration.

Meanwhile, the prefilled injectors generally have such a construction that a medicine is sealed between a plunger and a stopper fitted in a syringe provided at a tip end thereof with an injection nozzle. The prefilled injectors are operated such that when the plunger is pushed in the syringe to cause the stopper to move into a cavity portion located at the tip end of the syringe, the medicine is leaked out via bypass passages formed in the cavity portion along an outer periphery of the stopper, and then injected from the injection nozzle fitted at the tip end of the syringe. In the case where such prefilled injectors are sterilized or disinfected by steam or hot water, particular attention must be paid to prevent the deposition of water onto the injection nozzle or cap.

For example, in Japanese Patent Application Laid-Open (KOKAI) No. 8-308926(1996) (WO 96/28201), there are described a prefilled injector and a sterilizing method of the prefilled injector in which a syringe provided at a tip end thereof with a lure lock (needle-mounting portion) covered with a rubber protection cap is heated by steam while pressing the protection cap, thereby not only preventing the protection cap from being fallen off or dismounted by the expansion of air in a cavity portion located at the tip end of the syringe, but also inhibiting water drops from depositing onto an injection nozzle during the heat sterilization.

However, in the above-described conventional prefilled injectors, although the protection cap is fitted thereto upon the heat sterilization by steam, there still remains such a risk that water enters into the injection nozzle because of poor seal or looseness due to application of heat. Further, since an outer surface of the injector body is wetted upon the heat sterilization using steam, it is required to conduct a drying step in addition to the above two sterilization or disinfection treatments before packaging the injectors.

WO 94/13328 and US 6 228 324 provide a method and a packaging to sterilize an assembled and sealed prefilled delivery device and its content in its package.

As a result of the present inventors' earnest studies for solving the above problems, it has been found that a prefilled injector package comprising a prefilled injector having a syringe body filled with a medicine and a packaging material for hermetically packaging the prefilled injector such that the packaging material is brought into close contact with at least the syringe body, is capable of not only conducting simultaneous sterilization or disinfection of the injector body and a medicine filled therein by only one heat treatment because of excellent heat conductivity therefore when externally heated, but also preventing outside air containing water from entering thereinto, thereby upon the heat sterilization using steam or hot water, avoiding the deposition of water onto the injection body and keeping the injection nozzle in a substantially sterile condition. The present invention has been attained on the basis of this finding.

### SUMMARY OF THE INVENTION

The present invention seeks to provide a prefilled injector package capable of not only conducting simultaneous sterilization or disinfection of an injector body and a medicine filled therein by only one heat treatment, but also preventing the deposition of water onto an injector body, an injection nozzle or a cap when subjecting the injector package to sterilization using steam or hot water, and a method for sterilizing or disinfecting the prefilled injector package.

In a first aspect of the present invention, there is provided a prefilled injector package comprising a prefilled injector having a syringe body filled with a medicine and a packaging material for packaging the prefilled injector, wherein said prefilled injector is hermetically packaged by the packaging material such that the packaging material is brought into close contact with the syringe body over at least 75% of the surface area of the syringe body.

More specifically, the above prefilled injector package is hermetically packaged so that it is capable of simultaneous sterilization or disinfection of the injector body and the medicine filled therein by heat-treating the prefilled injector package by steam or hot water, while preventing water from depositing onto the injector body during the heat sterilization using steam or hot water.

In one specific embodiment, the injector is package by vacuum packaging or shrink packaging.

In a further specific embodiment the packaging material comprises a film having a gas-barrier property and/or a moisture proof property, preferably a multi-layered film having a gas-barrier property.

In a specific embodiment the packaging material comprises a film having an easy-peel property.

Preferably the medicine is viscoelastic. In a further preferred embodiment the medicine comprises one or more hyaluronic acid compounds.

In a specific embodiment the packaging material contacts the syringe body either directly or via a thermally-conductive layer.

In a second aspect of the present invention, there is provided a method for sterilizing or disinfecting the above prefilled injector package, wherein after packaging the prefilled injector having a syringe body containing a medicine, with a packaging material such that the packaging material is brought into close contact with the syringe body over at least 75% of the surface area of the syringe body, the prefilled injector package is heat-treated simultaneously to sterilize or to disinfect an injector body and the medicine contained therein.

More specifically, in the above sterilizing or disinfecting method, the prefilled injector package is packaged with the packaging material such that the packaging material comes into close contact with at least the syringe body, and then is heat-treated. Therefore, heat can be efficiently transmitted to the injector body and the medicine filled therein through the packaging material. Further, even when heat-treating the prefilled injector package by steam or hot water, the injector body is free from deposition of water thereonto.

### BRIEF DESCRIPTION OF THE DRAWINGS

Fig. 1 is a perspective view of a prefilled injector package according to the present invention.
Fig. 2 is a sectional view of a prefilled injector to be packaged to form the prefilled injector package of the present invention, which is taken along a longitudinal center line thereof.
Fig. 3 is a graph showing the change in temperature of the medicine filled in the injector body during sterilization by moist heat.

### DETAILED DESCRIPTION OF THE INVENTION

Next, one preferred embodiment of the present invention will be described in detail by referring to the accompanying drawings.

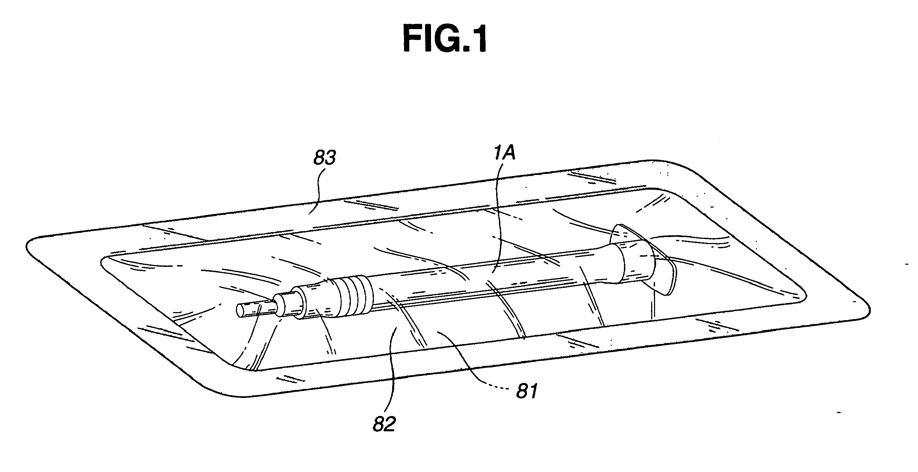

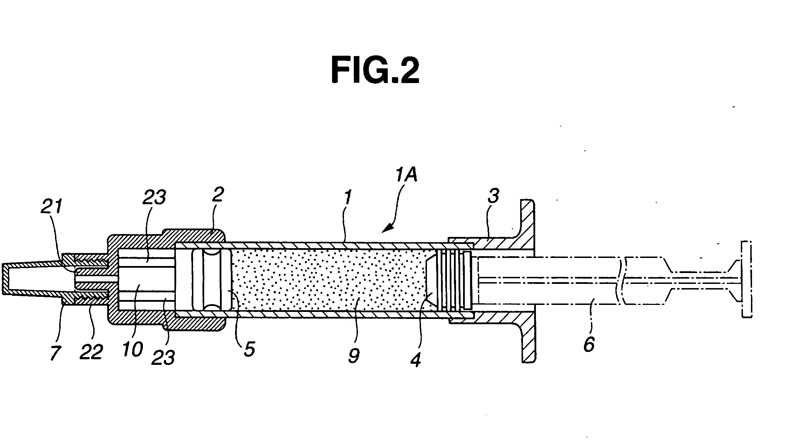

Fig. 1 shows a perspective view of a prefilled injector package according to the present invention; and Fig. 2 shows a sectional view of an example of a prefilled injector to be packaged to form the prefilled injector package of the present invention, which is taken along a longitudinal center line thereof.

First, the prefilled injector package of the present invention is explained. As shown in Fig. 1, the prefilled injector package of the present invention includes a prefilled injector and a packaging material for packaging the prefilled injector. As shown in Fig. 2, the prefilled injector of the present invention is a medical injector comprising a syringe (1A) having a syringe body (1) prefilled with a medicine (9). More specifically, a body of the injector is mainly constituted by the syringe (1A) provided at a tip end thereof with an injection nozzle (21) and a cylindrical lure lock (22) surrounding the injection nozzle, a medicine (9) sealed between a plunger (4) and a stopper (5) fitted in the syringe (1A), and a cavity portion (10) being provided at a tip side of the syringe (1A) and having a bypass passage structure. Meanwhile, in the prefilled injector to be packaged by the packaging material according to the present invention, the injector mainly constituted by the above all members except for the medicine filled therein is referred to as "injector body".

The medicine (9) to be filled in the syringe (1A) (i.e., the syringe body (1)) includes ordinary pharmaceutical products as well as liquid medicines and/or viscoelastic medicines to be classified into not pharmaceutical products but medical device. The properties and applications of such medicines (9) are not particularly restricted, and the medicines (9) are preferably those applicable to heat-sterilization. Examples of the medicines (9) may include solutions or gels of hyaluronic acid and modified products thereof (e.g., cross-linked hyaluronic acid as described in WO97/18244) which are used, e.g., as intra-articular injection agents, agents for ophthalmic surgery aid, agents for adhesion prevention for organs or tissues, etc. in various medical applications; collagen; high-viscoelastic medicines such as synthetic high polymers; or the like. In particular, hyaluronic acid and derivatives thereof are preferred for the purpose of the present invention because these medicines are highly-viscoelastic substances that are hard to be applicable to sterile filtration.

The syringe (1A) may be integrally formed by injection-molding synthetic resins. Alternatively, as shown in Fig. 2, the syringe (1A) may be formed by assembling the syringe body (1), an end member (2) and a finger-holding member (3). The syringe body (1) may be in the form of a cylindrical member made of transparent resin materials, typically those transparent resin materials having excellent chemical resistance and heat resistance such as cyclic polyolefins, polypropylene, polyethylene, poly(ethylene naphthalate), polycarbonates and ABS resins; glass; or the like.

The end member (2) is a member for installing, if required, with an injection needle or a tubular injection equipment such as a cannula or the like, and may be usually formed by injection-molding synthetic resins such as cyclic polyolefins, polypropylene, polyethylene, poly(ethylene naphthalate), polycarbonates and ABS resins. The end member (2) is constituted of a cylindrical shape having a substantially closed bottom, and is integrally formed at a tip end (bottom) thereof with the injection nozzle (21) and the lure lock (22). The end member (2) has, at a base side thereof (i.e., at the side close to the syringe body (1)), an inner diameter so as to allow the tip end of the syringe body (1) to be tightly interfitted thereinto. The end member (2) has, at the tip side thereof, an inner diameter substantially identical to or slightly larger than that of the syringe body (1) for forming the below-described bypass passage structure.

The injection nozzle (21) is provided at a tip end of the end member (2) along a longitudinal center line of the syringe (1A). The lure lock (22) is a mechanism for preventing falling-off of an injection equipment mounted to the injection nozzle (21), is formed to a cylindrical shape surrounding the injection nozzle (21), and is provided on an inner peripheral surface thereof with threads to which a treaded flange outwardly projecting from a base of the injection equipment can be screwed. The lure lock (22) is usually mounted with a cap (7) for protecting the lure lock as well as the injection nozzle (21) until use thereof. The finger-holding member (3) may be usually shaped a substantially cylindrical shape having two ear-shaped or jaw-shaped hooks outwardly extending in opposite directions from a rear end thereof. The finger-holding member (3) is mounted to the syringe body (1) by inserting the rear end of the syringe body thereinto.

The plunger (4) may be usually made of an elastic material such as rubbers and synthetic rubbers, and inserted into a substantially central portion or rear portion of the syringe body (1) so as to closely contact with the inner peripheral surface of the syringe body. The plunger (4) has, for example, a cylindrical body on an outer peripheral surface of which three ring-shaped packings are arranged along the center line thereof in order to improve its air-tightness to the inner peripheral surface of the syringe body (1). The plunger (4) is provided at its rear end with a plunger rod (6) for pushing the plunger into the syringe body. The plunger rod (6) is usually dismounted from the plunger (4), and mounted thereto upon use thereof.

The stopper (5) is a sealing member for sealing the medicine (9) previously filled in the syringe body (1), and may be made of an elastic material such as rubbers and synthetic rubbers similarly to the plunger (4). The stopper (5) is inserted into a front end portion of the syringe body (1) so as to closely contact with the inner peripheral surface of the syringe body. Such a stopper (5) has any suitable outer shape capable of being closely fitted with the inner peripheral surface of the syringe body (1), for example, a short-cylindrical shape having a slightly larger diameter at front and rear ends thereof.

The cavity portion (10) located at the tip side of the syringe (1A) is provided in the end member (2). The cavity portion (10) serves for accommodating the stopper (5), allowing the medicine (9) to bypass the stopper (5), and supplying the medicine (9) to the injection nozzle (21) when the medicine (9) is pushed out by the push-in operation of the plunger (4). The bypass passage structure provided in the cavity portion (10) for pushing out the medicine (9) therethrough may be in the form of a bypass flow path (23) constituted by a plurality of grooves formed on the inner peripheral surface of the tip side of the end member (2), or in the form of a clearance (not shown) formed between the inner peripheral surface of the end member (2) and the outer peripheral surface of the stopper (5) by enlarging the inner diameter of the tip side of the end member (2) as compared to that of the syringe body (1). Meanwhile, in order to more smoothly flow the medicine (9) from the bypass flow path (23) to the injection nozzle (21), projections (not shown) are preferably formed on the tip end surface of the stopper (5) or on the tip end inner surface of the end member (2).

As shown in Fig. 1, the prefilled injector package of the present invention includes the above-described injector and the packaging material for hermetically packaging the injector. In addition, according to the present invention, the packaging material is brought into close contact with at least the syringe body (1) (refer to Fig. 2). The packaging for allowing the packaging material to come into close contact with at least the syringe body can be accomplished, for example, by vacuum packaging or shrink packaging. The vacuum packaging is a packaging method of evacuating air from a sealed package in order to prevent deterioration in quality of goods enclosed therein, and may also be referred to as evacuation packaging or pressure-reduced packaging. Also, the shrink packaging is a packaging method of subjecting the packaging material to heat shrinkage in order to fixedly hold the goods enclosed therein.

The prefilled injector package shown in Fig. 1 is one of embodiments to which the vacuum packaging is applied. Such a prefilled injector package including, for example, a packaging material composed of two sheet-like films (81, 82) between which the injector is interposed, may be formed by welding overlapped outer peripheries of the films (81, 82) and evacuating air between the films, thereby packaging the injector. In the thus formed prefilled injector package, the films (81, 82) are brought into close contact with at least a substantially whole portion of the syringe body (1) of the syringe (1A). In Fig. 1, reference numeral (83) denotes a welded portion of the films (81, 82). Meanwhile, as long as the films are sealed around the injector body, it is not essential that the welded portion extends up to the edge of the outer periphery of the prefilled injector package.

As materials of the films (81, 82) as the packaging material, there may used those capable of hermetically packaging the prefilled injector. Specific examples of the packaging material may include weldable or heat-sealable thermoplastic resins such as polyethylene, polypropylene, poly(ethylene terephthalate) or the like. The films (81, 82) may be made of the same kind of resin, or may be in the form of a multi-layered film composed of two or more kinds of resins.

In particular, from the standpoint of good heat resistance upon the heat sterilization, it is preferred to use films having a high heat resistance. Also, from the standpoint of suitability for the heat sterilization using steam or hot water, it is preferred to use films having a high water-proof or moisture-proof property. The multi-layered film may be formed by suitably combining a plurality of film materials having the above respective properties. More specifically, the multi-layered film may be constituted from the combination of usual films made of polyethylene, polypropylene, poly(ethylene terephthalate), polyamides (nylons), poly(vinylidene chloride) or the like.

The above multi-layered film may also be in the form of a laminated film composed of a weldable thermoplastic resin layer and a gas-barrier resin layer having a gas-barrier property laminated thereon. Examples of the gas-barrier resin may include ethylene-vinyl alcohol copolymer, polyamides, poly(ethylene naphthalate), poly(vinylidene chloride) or the like. Further, in order to enhance a heat resistance and a gas-barrier property, aluminum-deposited films can also be used. When the multi-layered film having a gas-barrier property is used as the packaging material, oxygen and water can be prevented from penetrating therethrough, thereby keeping the injector body enclosed therein in unstained and hygienic conditions.

Also, the films (81, 82) may be made of different kinds of resins, for example, one may be a transparent film made of polyethylene, polypropylene or poly(ethylene terephthalate), and the other may be an opaque film such as an aluminum-deposited film. Further, in order to facilitate the opening of the prefilled injector package, the packaging material may be constituted from films provided with cuts such as V notches, or peel-openable films. In order to achieve peel-openable packaging, there may be used ordinary multi-layered films having an easy-peel property and a releasing property. Examples of such peel-openable multi-layered films may include those having a polypropylene-modified sealant layer as an innermost layer.

The method for producing the above prefilled injector package, which includes the order of assembly thereof, is not particularly restricted. In general, the prefilled injector may be produced by inserting the stopper (5) into the syringe (1A), fitting the cap (7) onto the injection nozzle (21) and the lure lock (22) provided at a tip end of the syringe (1A), filling the medicine (9) in the syringe (1A), and then inserting the plunger (4) into the syringe (1A). The thus produced prefilled injector is then set in a vacuum packaging apparatus to form a sealed prefilled injector package as shown in Fig. 1.

As described above, since the packaging material is brought into close contact with at least the syringe body (1), the resultant prefilled injector package is excellent in heat conductivity when externally heated. Therefore, not only the injector body but also the medicine (9) filled in the syringe (1A) can be sterilized or disinfected at the same time by only one heat treatment, resulting in reduction of production costs. The wording: "disinfection" used in the present invention means that germs and/or bacteria in the prefilled injector package are killed such that the treated prefilled injector package enables to be brought into and used in an operating room.

In addition, in the prefilled injector package of the present invention, since the prefilled injector is packaged in a hermetically sealed condition, water is prevented from entering thereinto. Therefore, if the prefilled injector package is heat-sterilized using steam or hot water, water is prevented from depositing onto the injector body, so that the injection nozzle (21) and the lure lock (22) can be kept in a substantially sterile condition. Further, the drying step conventionally required before packaging can be omitted. Also, the prefilled injector package of the present invention is formed into a flat sheet-like shape as a whole as shown in Fig. 1, and, therefore, is less bulky and advantageous for storage and transportation, resulting in reduction of distributing or marketing costs. In addition, since the amount of the packaging material is reduced, the disposal treatment thereof after use can be facilitated.

Meanwhile, in the present invention, as the prefilled injector to be packaged, there may be used, in addition to the above prefilled injectors having the construction as shown in Fig. 2, other prefilled injectors having various constructions such as those described in Japanese Patent No. 3190988 (WO 92/06722) and Japanese Patent Application Laid-Open (KOKAI) No. 10-155905(1998) (USP 6,126,644) as well as those usually used as medical goods for pharmaceutical products or medical devices. Also, the plunger rod of the injector body may be fitted to the plunger after opening the prefilled injector package upon use as described above, or the injector body fitted with the plunger rod may be packaged according to the present invention.

Next, the method for sterilizing or disinfecting the prefilled injector package according to the present invention is explained. The sterilizing or disinfecting method of the above prefilled injector package according to the present invention comprises the steps of packaging the prefilled injector at a late stage of the production process of the prefilled injector package such that the packaging material is brought into close contact with at least the syringe body (1) as described above, and then heat-treating the thus formed prefilled injector package to sterilize or disinfect the injector body and the medicine (9) filled therein at the same time.

That is, the above prefilled injector package is placed in a heater such as autoclave, and subjected to heat sterilization therein using steam or hot water. In the present invention, the "heat sterilization" means a heat treatment such as sterilization by moist heat using steam or hot water in which at least the medicine filled in the injector body is heated and sterilized. For example, the above sterilization by moist heat may be conducted at 121°C for 20 minutes when using a heater such as autoclave.

According to the sterilizing or disinfecting method of the present invention, it is possible to sterilize or disinfect the medicine (9) filled in the syringe (1A) and the injector body at the same time by subjecting the prefilled injector package to only one heat treatment subsequent to the packaging process. By this heat treatment, at least the medicine (9) can be sterilized. Moreover, the prefilled injector package treated by the sterilizing or disinfecting method of the present invention can be brought into and used in an operating room. In other words, by subjecting the packaged injector as marketable goods to only one heat treatment, it is possible to sterilize or disinfect the medicine (9), the injector body and an inside of the packaging material (i.e., an inside one of the films (81, 82)), thereby producing the prefilled injector package in an extremely efficient manner.

Furthermore, according to the sterilizing or disinfecting method of the present invention, since the prefilled injector is packaged in a hermetically sealed condition and then subjected to the heat sterilization, water is prevented from entering into the prefilled injector package, so that the injector body including the injection nozzle (21), the cap (7) and the lure lock (22) are free from deposition of water thereonto. Also, when the films especially having excellent moisture-proof property and gas-barrier property are selectively used as the packaging material, the deposition of water can be more surely prevented.

As explained above, since the prefilled injector package of the present invention shows an excellent heat conductivity when externally heated, it is possible to sterilize or disinfect the medicine and the injector body at the same time by subjecting the prefilled injector package to only one heat treatment. Further, since outside air containing water is prevented from entering into the injector package, the deposition of water onto the injection body can be prevented even though the prefilled injector package is subjected to heat sterilization using steam or hot water, thereby keeping the injection nozzle in a substantially sterile condition.

In the method for sterilizing or disinfecting the prefilled injector package according to the present invention, the injector body and the medicine filled therein can be sterilized or disinfected at the same time or at least the medicine can be sterilized by subjecting the prefilled injector package to only one heat treatment subsequent to the packaging process, thereby the injector package can be produced in an extremely efficient manner. Besides, since the injector is packaged in a hermetically sealed condition and then heat-sterilized, the deposition of water onto the injector body including the injection nozzle can be effectively prevented.

### EXAMPLE

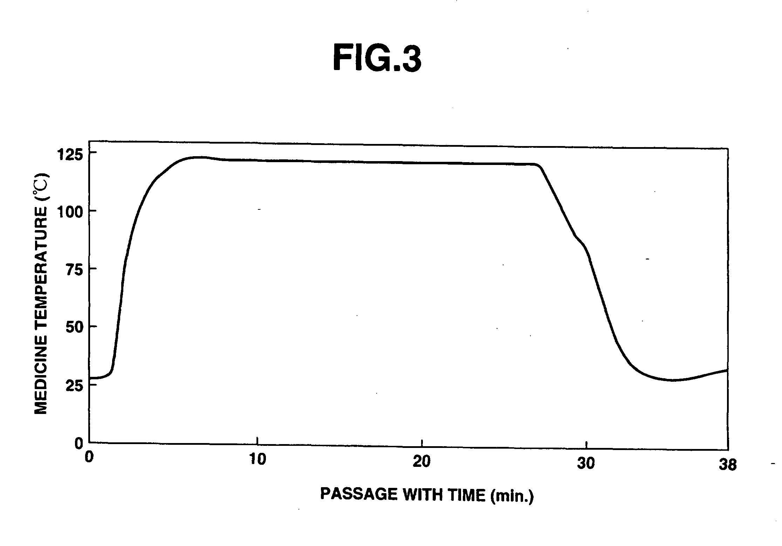

The present invention is described in more detail by the following example. Fig. 3 is a graph showing the change in temperature of the medicine filled in the injector during the sterilization by moist heat.

First, an injector having an inner capacity of 5 ml (injector body having such a construction as shown in Fig. 2) was filled with about 5 ml of a solution of a hyaluronic acids compound as a medicine, to produce a prefilled injector. Then, the thus produced prefilled injector was enclosed in a packaging bag made of a multi-layered film comprising poly(ethylene terephthalate)/nylon/polypropylene, and packaged by vacuum packaging method, thereby producing a prefilled injector package. Meanwhile, in the vacuum packaging, a temperature sensor for measuring a temperature of the medicine filled in the injector in the subsequent sterilization process was inserted into one medicine-receiving chamber (syringe body) from the plunger side thereof, and the prefilled injector equipped with the temperature sensor was packaged to produce the prefilled injector package. The thus produced prefilled injector package including the temperature sensor was placed in an autoclave, and subjected therein to sterilization by moist heat at 121°C for about 30 minutes.

After completion of the sterilization by moist heat in the autoclave, the prefilled injector was taken out from the bag and visually observed to examine the condition thereof. As a result, it was confirmed that the injector body including the injection nozzle, cap and lure lock was free from deposition of water thereonto. Also, as a result of the measurement of the medicine temperature during the sterilization by moist heat, it was confirmed that the medicine temperature was changed as shown in Fig. 3. That is, it was confirmed that the medicine was maintained at a temperature of not less than 121°C for a period of not less than 20 minutes and, therefore, could be sufficiently heated.

## Claims

1. A prefilled injector package comprising:
a prefilled injector having a syringe body (a) filled with a medicine (9); and
a packaging material for packaging the prefilled injector,
wherein said prefilled injector is hermetically packaged by the packaging material (81, 82) **characterised in that** the packaging material is brought into close contact with the syringe body over at least 75% of the surface area of the syringe body.

2. A package according to claim 1, wherein said injector is packaged by vacuum packaging or shrink packaging.

3. A package according to claim 1 or 2, wherein said packaging material (81, 82) comprises a film having a gas-barrier property and/or a moisture-proof property.

4. A package according to any preceding claim, wherein said packaging material (81, 82) comprises a multi-layered film having a gas-barrier property.

5. A package according to any preceding claim, wherein said packaging material (81, 82) comprises a film having an easy-peel property.

6. A package according to any preceding claim, wherein said medicine (9) is viscoelastic.

7. A package according to any preceding claim, wherein said medicine (9) comprises one or more hyaluronic acid compound.

8. A package according to any preceding claim, wherein said close contact consists in said packaging material (81, 82) contacting said syringe body (1) either directly or via a thermally-conductive layer.

9. A method for sterilizing or disinfecting the prefilled injector package according to any preceding claim, wherein after packaging the prefilled injector having a syringe body (1) containing a medicine (9), with a packaging material (81, 82) such that the packaging material is brought into close contact with the syringe body over at least 75% of the surface area of the syringe body, the prefilled injector package is heat-treated simultaneously to sterilize or to disinfect an injector body and the medicine contained therein.

## Patentansprüche

1. Vorgefüllte Injektorpackung, umfassend:
einen vorgefüllten Injektor, der einen Spritzenkörper (a), gefüllt mit einer Medizin (9), aufweist; und
ein Verpackungsmaterial zum Verpacken des vorgefüllten Injektors,
worin der vorgefüllte Injektor durch das Verpackungsmaterial (81, 82) hermetisch verpackt ist, **dadurch gekennzeichnet, daß** das Verpackungsmaterial in engen Kontakt mit dem Spritzenkörper über zumindest 75 % der Oberfläche des Spritzenkörpers gebracht ist.

2. Packung gemäß Anspruch 1, worin der Injektor durch Vakuumverpackung oder Schrumpfverpackung verpackt ist.

3. Packung gemäß Anspruch 1 oder 2, worin das Verpackungsmaterial (81, 82) eine Folie umfaßt, die Gasbarriereneigenschaft und/oder eine feuchtigkeitabdichtende Eigenschaft aufweist.

4. Packung gemäß irgendeinem vorhergehenden Anspruch, worin das Verpackungsmaterial (81, 82) eine Multischichtfolie mit Gasbarriereneigenschaft umfaßt.

5. Packung gemäß irgendeinem vorhergehenden Anspruch, worin das Verpackungsmaterial (81, 82) eine Folie mit leichter Ablöseeigenschaft ("easy-peel property") umfaßt.

6. Packung gemäß irgendeinem vorhergehenden Anspruch, worin die Medizin (9) viskoelastisch ist.

7. Packung gemäß irgendeinem vorhergehenden Anspruch, worin die Medizin (9) eine oder mehrere Hyaluronsäureverbindung(en) umfaßt.

8. Packung gemäß irgendeinem vorhergehenden Anspruch, worin der enge Kontakt darin besteht, daß das Verpackungsmaterial (81, 82) den Spritzenkörper (1) kontaktiert, entweder direkt oder über eine thermisch leitende Schicht.

9. Verfahren zum Sterilisieren oder Desinfizieren der vorgefüllten Injektorpackung gemäß irgendeinem vorhergehenden Anspruch, worin nach dem Verpacken des vorgefüllten Injektors, der einen Spritzenkörper (1), der eine Medizin (9) enthält, aufweist, mit einem Verpackungsmaterial (81, 82), so daß das Verpackungsmaterial in engen Kontakt mit dem Spritzenkörper über zumindest 75 % der Oberfläche des Spritzenkörpers gebracht wird, die vorgefüllte Injektorpackung simultan wärmebehandelt wird, um den Injektorkörper und die hierin enthaltene Medizin zu sterilisieren oder zu desinfizieren.

## Revendications

1. Emballage d'injecteur pré-rempli comprenant :
un injecteur pré-rempli ayant un corps de seringue (a) rempli d'un médicament (9); et
un matériau d'emballage pour emballer l'injecteur pré-rempli,
dans lequel ledit injecteur pré-rempli est emballé hermétiquement grâce au matériau d'emballage (81, 82), **caractérisé en ce que** le matériau d'emballage est mis en contact étroit avec le corps de seringue sur au moins 75 % de la surface du corps de seringue.

2. Emballage selon la revendication 1, dans lequel ledit injecteur est emballé par emballage sous vide ou emballage par rétraction.

3. Emballage selon la revendication 1 ou 2, dans lequel ledit matériau d'emballage (81, 82) comprend un film ayant une propriété de barrière contre les gaz et/ou une propriété de résistance à l'humidité.

4. Emballage selon l'une quelconque des revendications précédentes, dans lequel ledit matériau d'emballage (81, 82) comprend un film multicouche ayant une propriété de barrière contre les gaz.

5. Emballage selon l'une quelconque des revendications précédentes, dans lequel ledit matériau d'emballage (81, 82) comprend un film ayant une propriété d'ouverture facile.

6. Emballage selon l'une quelconque des revendications précédentes, dans lequel ledit médicament (9) est viscoélastique.

7. Emballage selon l'une quelconque des revendications précédentes, dans lequel ledit médicament (9) comprend un ou plusieurs composé(s) d'acide hyaluronique.

8. Emballage selon l'une quelconque des revendications précédentes, dans lequel ledit contact étroit consiste en ce que ledit matériau d'emballage (81, 82) est en contact avec ledit corps de seringue (1) soit directement, soit par le biais d'une couche thermoconductrice.

9. Procédé de stérilisation ou de désinfection de l'emballage d'injecteur pré-rempli selon l'une quelconque des revendications précédentes, dans lequel après avoir emballé l'injecteur pré-rempli ayant un corps de seringue (1) contenant un médicament (9), avec un matériau d'emballage (81, 82) de telle sorte que le matériau d'emballage soit mis en contact étroit avec le corps de seringue sur au moins 75 % de la surface du corps de seringue, l'emballage d'injecteur pré-rempli est traité thermiquement simultanément pour stériliser ou pour désinfecter un corps d'injecteur et le médicament contenu dans celui-ci.
